# EUROPEAN PATENT SPECIFICATION

(11) **EP 2 063 951 B1**
(45) Date of publication and mention of the grant of the patent: **22.05.2019**
(21) Application number: 07842423.1
(22) Date of filing: 13.09.2007
(51) Int. Cl.: A61M 25/10, A61M 25/00, A61M 29/02

(54) **BALLOON CATHETER**
BALLONKATHETER
SONDE À BALLONNET

(30) Priority: 13.09.2006 US 825551 P
(43) Date of publication of application: 03.06.2009
(73) Proprietor: Boston Scientific Limited, Hamilton HM11 (BM)
(72) Inventor: GOODIN, Richard, St. Cloud, MN 56304 (US)
(74) Representative: Vossius & Partner Patentanwälte Rechtsanwälte mbB
(86) International application number: PCT/US2007/078392
(87) International publication number: WO 2008/033998

(56) References cited:
- EP-A- 0 998 955
- WO-A-93/01856
- US-A1- 2007 151 650

## Description

### Related Applications

This application claims priority to U.S. Provisional Application Serial No. 60/825,551 filed September 13, 2006.

### Technical Field

The present invention relates generally to medical devices and more particularly to balloon catheters. Such a device is disclosed in the WO9301856.

### Background

Heart and vascular disease are major problems in the United States and throughout the world. Conditions such as atherosclerosis result in blood vessels becoming blocked or narrowed. This blockage can result in lack of oxygenation of the heart, which has significant consequences since the heart muscle must be well oxygenated in order to maintain its blood pumping action.

Occluded, stenotic, or narrowed blood vessels may be treated with a number of relatively non-invasive medical procedures including percutaneous transluminal angioplasty (PTA), percutaneous transluminal coronary angioplasty (PTCA), and atherectomy. Angioplasty techniques typically involve the use of a balloon catheter. The balloon catheter is advanced over a guidewire so that the balloon is positioned adjacent a stenotic lesion. The balloon is then inflated, and the restriction of the vessel is opened.

There is an ongoing need for improved angioplasty devices, including balloon catheters.

### Summary

The invention as further disclosed in claim 1 pertains to improved medical devices providing advantages in flexibility, strength and other desired properties.

Accordingly, an example embodiment of the disclosure may be found in a balloon catheter that includes an elongate shaft and a shaft lumen extending through the elongate shaft to a distal end thereof. A balloon may be disposed about a distal region of the elongate shaft while a coil may extend through at least part of the balloon. A polymer coating may be disposed on an interior of the balloon.

Another example embodiment of the disclosure may be found in a balloon catheter that includes an elongate shaft and a shaft lumen extending through the elongate shaft to a distal end thereof. A distal guidewire port may be disposed at or near the distal end of the elongate shaft. A balloon may be disposed about a distal region of the elongate shaft. A proximal guidewire port may be disposed within the distal region of the elongate shaft at a position that is proximal of the balloon. A coil may be disposed within the balloon and may include or be formed from a coil wire that is polymer coated prior to being coiled.

Another example embodiment of the disclosure may be found in a method of forming a balloon catheter that includes an elongate shaft, a balloon disposed about the elongate shaft and a coil disposed within the elongate shaft. A flat ribbon coil wire may be coated with a fluoropolymer and may subsequently be coiled to form a coil. A heat shrink wrap may be heated onto an exterior of the coil. The coil may be secured inside the elongate shaft and the balloon may be secured about the elongate shaft.

The above summary of the present invention is not intended to describe each disclosed embodiment or every implementation of the present invention. The Figures, Detailed Description and Examples which follow more particularly exemplify these embodiments.

### Brief Description of the Figures

The invention may be more completely understood in consideration of the following detailed description of various embodiments of the invention in connection with the accompanying drawings, in which:
Figure 1 is a schematic view of an illustrative but non-limiting balloon catheter in accordance with the present invention;
Figure 2 is a schematic partial cross-sectional view of a portion of an illustrative but non-limiting balloon catheter not in accordance with the present invention;
Figure 3 is a schematic partial cross-sectional view of a portion of an illustrative but non-limiting balloon catheter in accordance with the present invention; and
Figure 4 is a schematic partial cross-sectional view of a portion of an illustrative but non-limiting balloon not in accordance with the present invention.

While the invention is amenable to various modifications and alternative forms, specifics thereof have been shown by way of example in the drawings and will be described in detail. It should be understood, however, that the intention is not to limit the invention to the particular embodiments described. On the contrary, the intention is to cover all modifications, equivalents, and alternatives falling within the scope of the invention.

### Detailed Description

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g., 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following description should be read with reference to the drawings wherein like reference numerals indicate like elements throughout the several views. The drawings, which are not necessarily to scale, depict illustrative embodiments of the claimed invention.

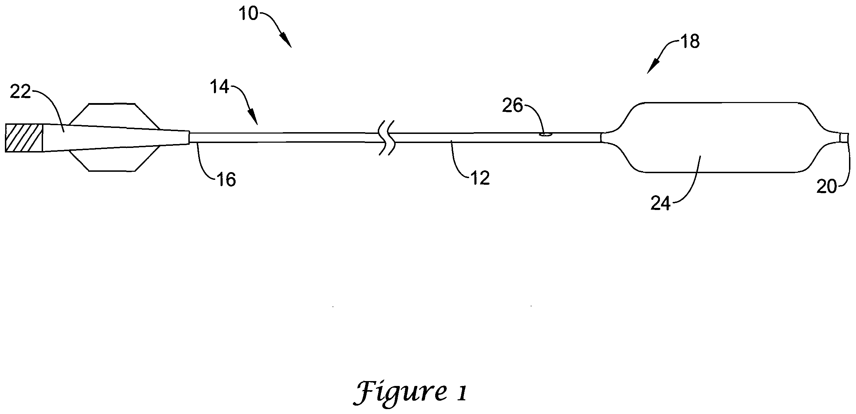

Figure 1 is a plan view of a catheter 10 The catheter 10 can be any of a variety of different catheters. In some embodiments, the catheter 10 can be an intravascular catheter. Examples of intravascular catheters include balloon catheters, atherectomy catheters, drug delivery catheters, stent delivery catheters, diagnostic catheters and guide catheters. The intravascular catheter 10 can be sized in accordance with its intended use. The catheter 10 can have a length that is in the range of about 100 to 150 centimeters and can have any useful diameter. As illustrated, Figure 1 portrays a balloon catheter, but the invention is not limited to such. Except as described herein, the intravascular catheter 10 can be manufactured using conventional techniques.

In the illustrated embodiment, the intravascular catheter 10 includes an elongate shaft 12 that has a proximal region 14 defining a proximal end 16 and a distal region 18 defining a distal end 20. A hub and strain relief assembly 22 can be connected to the proximal end 16 of the elongate shaft 12. The hub and strain relief assembly 22 can be of conventional design and can be attached using conventional techniques. It is also recognized that alternative hub designs can be incorporated into embodiments of the present invention.

The elongate shaft 12 can include one or more shaft segments having varying degrees of flexibility. For example, the elongate shaft may include a relatively stiff proximal portion, a relatively flexible distal portion and an intermediate position disposed between the proximal and distal portions having a flexibility that is intermediate to both.

In some cases, the elongate shaft 12 or portions thereof may be formed of a single polymeric layer. In some instances, the elongate shaft 12 may include an inner liner such as an inner lubricious layer and an outer layer. In some cases, the elongate shaft 12 may include a reinforcing braid layer disposed between the inner and outer layers. The elongate shaft 12 is considered herein as generically representing a catheter to which various elements can be added to provide the catheter 10 with adjustable stiffness.

If the elongate shaft 12 includes an inner liner, the inner liner can include or be formed from a coating of a material having a suitably low coefficient of friction. Examples of suitable materials include perfluoro polymers such as polytetrafluoroethylene (PTFE), better known as TEFLON®, high density polyethylene (HDPE), polyarylene oxides, polyvinylpyrolidones, polyvinylalcohols, hydroxy alkyl cellulosics, algins, saccharides, caprolactones, and the like, and mixtures and combinations thereof.

The elongate shaft 12 can include, as an outer layer or layers, any suitable polymer that will provide the desired strength, flexibility or other desired characteristics. Polymers with low durometer or hardness can provide increased flexibility, while polymers with high durometer or hardness can provide increased stiffness. In some embodiments, the polymer material used is a thermoplastic polymer material. Some examples of suitable materials include polyurethane, elastomeric polyamides, block polyamide/ethers (such as PEBAX®), silicones, and co-polymers. The outer polymer layer can be a single polymer, multiple longitudinal sections or layers, or a blend of polymers. In some instances, a thermoplastic polymer such as a co-polyester thermoplastic elastomer, for example, available commercially under the ARNITEL® name, can be used.

In some instances, elongate shaft 12 or portions thereof may include or be formed from one or more metallic materials. In some cases, metals may be used in combination with one or more polymers such as those discussed above. Examples of suitable metals for inclusion in part or all of elongate shaft 12 include stainless steel, such as 300 series stainless steel (including 304V, 304L, and 316L; 400 series martensitic stainless steel; tool steel; nickel-titanium alloy such as linear-elastic or super-elastic Nitinol, nickel-chromium alloy, nickel-chromium-iron alloy, cobalt alloy, tungsten or tungsten alloys, MP35-N (having a composition of about 35% Ni, 35% Co, 20% Cr, 9.75% Mo, a maximum 1% Fe, a maximum 1% Ti, a maximum 0.25% C, a maximum 0.15% Mn, and a maximum 0.15% Si), hastelloy, monel 400, inconel 825, or the like; or other suitable material.

The catheter 10 also includes an inflatable balloon 24 that is disposed about the elongate shaft 12 within the distal region 18 thereof. The balloon 24 may be made from typical angioplasty balloon materials including polymers such as polyethylene terephthalate (PET), polyetherimide (PEI), polyethylene (PE), etc. Some other examples of suitable polymers, including lubricious polymers, may include polytetrafluoroethylene (PTFE), ethylene tetrafluoroethylene (ETFE), fluorinated ethylene propylene (FEP), polyoxymethylene (POM), polybutylene terephthalate (PBT), polyether block ester, polyurethane, polypropylene (PP), polyvinylchloride (PVC), polyether-ester (for example, and a polyether-ester elastomer such as ARNITEL® available from DSM Engineering Plastics).

Additional examples of suitable polymers include polyester (for example, a polyester elastomer such as HYTREL® available from DuPont), polyamide (for example, DURETHAN® available from Bayer or CRISTAMID® available from Elf Atochem), elastomeric polyamides, block polyamide/ethers, nylons such as polyether block amide (PEBA, for example, available under the trade name PEBAX®), silicones, Marlex high-density polyethylene, Marlex low-density polyethylene, linear low density polyethylene (for example, REXELL®), polyetheretherketone (PEEK), polyimide (PI), polyphenylene sulfide (PPS), polyphenylene oxide (PPO), polysulfone, nylon, perfluoro(propyl vinyl ether) (PFA), other suitable materials, or mixtures, combinations, copolymers thereof, polymer/metal composites, and the like.

In some cases, it may be desirable to use high modulus or generally stiffer materials so as to reduce balloon elongation. The above list of materials includes some examples of higher modulus materials. Some other examples of stiffer materials include polymers blended with liquid crystal polymer (LCP) as well as the materials listed above. For example, the mixture can contain up to about 5% LCP.

In some cases, the catheter 10 may represent an over-the-wire (OTW) catheter in which a guidewire (not illustrated) may, in use, enter the catheter 10 via proximal hub 22 and may exit through an opening at the distal end 20. In some instances, the catheter 10 may represent a rapid-exchange, or single-operator-exchange catheter (SOE) in which a guidewire may pass through a shorter guidewire lumen extending proximally from the distal end 20 to a guidewire port 26. In some cases, it will be recognized that the catheter 10 may be adapted to accommodate both OTW and SOE operation.

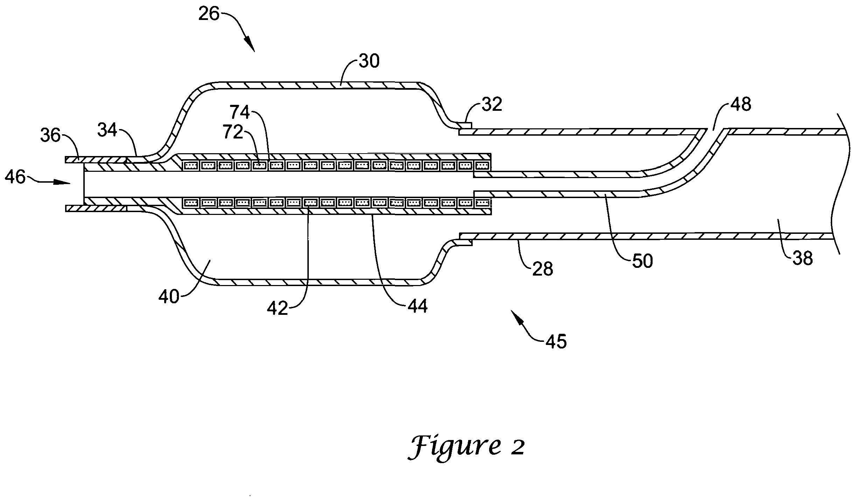

Figure 1 provides an overview of the catheter 10. Figures 2 through 5 provide greater detail regarding features of the catheter 10, particularly within the distal region 18 thereof. Figure 2 is a schematic cross-sectional view of a distal portion of a catheter 26 that, aside from features specifically discussed with respect to Figure 2, shares many features in common with the catheter 10 discussed with respect to Figure 1.

The catheter 26 includes an elongate shaft 28 that may be constructed in accordance with the materials discussed previously with respect to elongate shaft 12 (Figure 1). A balloon 30 having a proximal waist 32 and a distal waist 34 is disposed about the elongate shaft 28, and may be secured to the elongate shaft 28 using any suitable technique such as laser welding, thermal bonding, adhesive and the like. The balloon 30 may be formed of any suitable polymeric materials, such as those described with respect to Figure 1. In some cases, the balloon 30 may include or be formed from a polyamide such as PEBAX® 7233.

If desired, a bumper tip 36 may be secured distal of the balloon 30, but this is not required. If present, the bumper tip 36 may be formed of any suitably soft polymeric material to reduce potential tissue damage.

The elongate shaft 28 includes an inflation lumen 38 that is in fluid communication with an interior 40 of the balloon 30 and that can be used to provide sufficient inflation fluid (saline or the like) to inflate or deflate the balloon 30 as desired. The elongate shaft 28 may be formed of any suitable metallic or polymeric material such as those discussed with respect to Figure 1. In some instances, the elongate shaft 28 may include or be formed from a polyamide such as PEBAX®. In particular instances, the elongate shaft 28 may include or be formed from PEBAX® 7033.

Disposed within the interior 40 of the balloon 30 is a coil 42 that is covered by a polymer sheath 44. In many cases, the polymer sheath 44 is a polyamide heat shrink tube that has been, via application of heat and/or pressure, shrunk down onto the coil 42. The polymer sheath 44 may, in some instances, include or be formed from a polyamide such as PEBAX® having a durometer ranging from about 40D to about 70D. In particular cases, the polymer sheath 44 may be formed of a PEBAX® having a durometer of about 63D.

In some instances, as illustrated, the polymer sheath 44 may extend all the way to a distal end 46 of the catheter 26. It will be recognized that in forming the catheter 26, a mandrel (not illustrated) may be temporarily inserted into the distal end of the polymer sheath 44 prior to shrinking the polymer to retain a guidewire lumen therethrough.

In some cases, the polymer sheath 44 may not extend all the way to the distal end 46, but may extend at least to a position proximate the distal waist 34. As a result, the combination of the coil 42 and the polymer sheath 44 may provide a fluid-tight passage through the balloon 30 such that a guidewire (not illustrated) may pass through while not interfering with an ability to inflate and/or deflate the balloon 30 as desired.

In some instances, the coil 42 may be formed from a coil wire that has been polymer coated before the coil wire is wrapped or coiled into a coil form. In some cases, the coil wire is coated with a fluoropolymer such as polytetrafluoroethylene prior to being coiled into the coil 42. This can result in a coil 42 that has a fluoropolymer coating on an interior of the coil 42. This provides reduced friction for any guidewire advanced through the coil 42.

In some cases, it is contemplated that other techniques may be used to provide a fluoropolymer coating on an interior of the coil 42. In some instances, the fluoropolymer coating may be applied via sputter coating or dip coating, for example.

Any suitable coil wire may be used to form the coil 42. In some cases, the coil 42 may be formed from a flat ribbon coil wire having a relatively flat rectangular profile. In some instances, the flat ribbon coil wire may have a width-to-height ratio of about 3:1, about 4:1, about 5:1, about 6:1, about 7:1 or even wider. In particular cases, the flat ribbon coil wire may, for example, have a cross-sectional width of about 0,1778mm (seven thousands of an inch) and a height of about 0,0254mm (1 thousands of an inch). The coil wire used to form the coil 42 may be formed of any polymeric or metallic material, such as those materials recited above. In particular instances, the coil 42 may be formed from a stainless steel coil wire. It can be seen that each individual coil turning 72 is at least substantially if not completely coated with a polymeric coating 74.

As illustrated, the catheter 26 is adapted to provide SOE functionality. The catheter 26 includes a guidewire port 48 that leads to a guidewire lumen 50. The guidewire port 48 is disposed within a distal region 45 of the catheter 26 but is proximal of the balloon 30. In some instances, the polymer sheath 44 covering the coil 42 extends proximally a sufficient distance to form a fluid-tight seal with the guidewire lumen 50. As a result, the guidewire port 48 does not interfere with an ability to inflate and/or deflate the balloon 30 as desired. In some cases, the polymer sheath 44 is heat-shrunk onto the guidewire lumen 50. In some instances, the guidewire lumen 50 may instead be adhesively secured to the coil 42 and/or the polymer sheath 44.

The guidewire lumen 50 may be formed in any suitable manner and of any suitable material. In some cases, the guidewire lumen 50 may be formed by extending a polymeric tube between the coil 42 and the guidewire port 48. In some instances, the guidewire lumen 50 may be a metallic construct. In many instances, the construction shown within the distal portion of the catheter 26 may provide for improved pushability and improved flexibility all the way to the distal end 46.

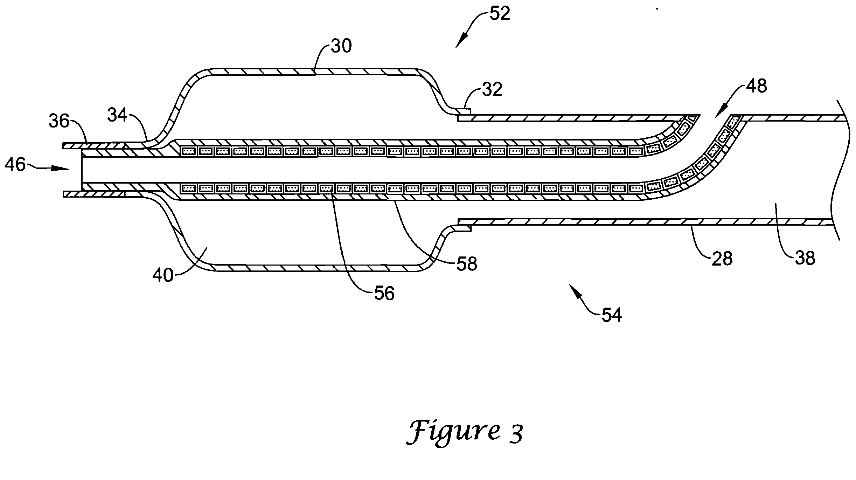

Figure 3 is a schematic cross-sectional view of a distal portion of a catheter 52 that, aside from features specifically discussed with respect to Figure 3, shares many features in common with the catheter 10 discussed with respect to Figure 1 as well as the catheter 26 discussed with respect to Figure 2.

The catheter 52 is also an SOE catheter, having a guidewire port 48 that is disposed within a distal region 54 of the catheter 52 but is proximal of the balloon 30. The catheter 52 includes a coil 56 that extends from a position within the balloon 30 at or near the distal waist 34 all the way to the guidewire port 48. A polymer sheath 58 extends at least from a position at or near the distal waist 34 (if it does not extend all the way to the distal end 46) to the guidewire port 48.

The coil 56 and the polymer sheath 58 are formed in a manner analogous to that described with respect to the coil 42 and the polymer sheath 44, respectively, of Figure 2. In some instances, the coil 56 is formed of a flat ribbon coil wire such as stainless steel that has been coated with a fluoropolymer such as polytetrafluoroethylene prior to coiling. In some cases, the polymer sheath 58 may, in some instances, include or be formed from a polyamide such as PEBAX® having a durometer ranging from about 40D to about 70D.

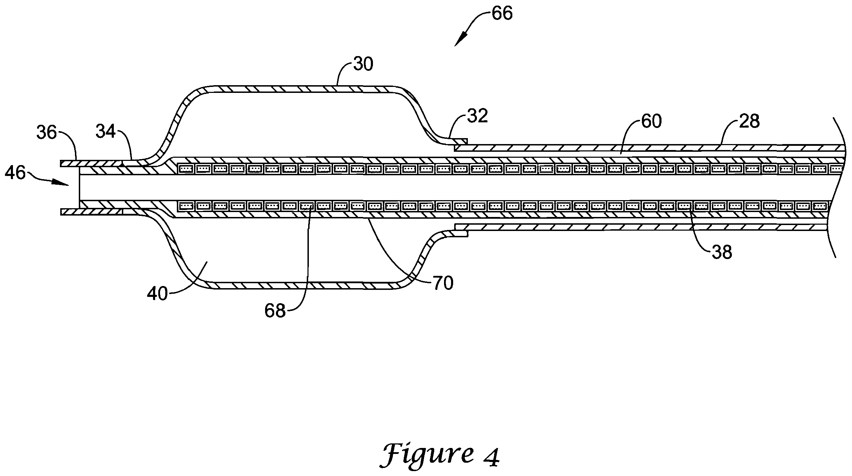

Figure 4 illustrates an OTW catheter 66 having a coil 68 and a polymer sheath 70. As illustrated, the coil 68 and the polymer sheath 70 extend proximally an indefinite distance. In some cases, the coil 68 and the polymer sheath 70 may extend proximally only about as far as the balloon 30 extends. In some instances, the coil 68 and the polymer sheath 70 may extend all the way to a proximal hub (not illustrated), or may terminate at some intermediate position. An annular inflation lumen 60 extends between the elongate shaft 28 and the polymer sheath 70.

The coil 68 is constructed in accordance with the materials and techniques discussed with respect to the coil 42. Similarly, the polymer sheath 70 is constructed in accordance with the materials and techniques discussed with respect to the polymer sheath 44. In some instances, the coil 68 is formed of a flat ribbon coil wire such as stainless steel that has been coated with a fluoropolymer such as polytetrafluoroethylene prior to coiling. In some cases, the polymer sheath 70 may, in some instances, include or be formed from a polyamide such as PEBAX® having a durometer ranging from about 40D to about 70D.

In some embodiments, part or all of the devices described herein can include a lubricious coating. Lubricious coatings can improve steerability and improve lesion crossing capability. Examples of suitable lubricious polymers include hydrophilic polymers such as polyarylene oxides, polyvinylpyrolidones, polyvinylalcohols, hydroxy alkyl cellulosics, algins, saccharides, caprolactones, and the like, and mixtures and combinations thereof. Hydrophilic polymers can be blended among themselves or with formulated amounts of water insoluble compounds (including some polymers) to yield coatings with suitable lubricity, bonding, and solubility. In some embodiments, portions of the devices described herein can be coated with a hydrophilic polymer or a fluoropolymer such as polytetrafluoroethylene (PTFE), better known as TEFLON®.

The invention should not be considered limited to the particular examples described above, but rather should be understood to cover all aspects of the invention as set out in the attached claims. Various modifications, equivalent processes, as well as numerous structures to which the invention can be applicable will be readily apparent to those of skill in the art upon review of the instant specification.

## Claims

1. A balloon catheter (52), comprising:
an elongate shaft (28) having a distal region (54) defining a distal end, a shaft lumen extending through the elongate shaft to the distal end;
a balloon (30) disposed about the distal region of the elongate shaft;
a coil (56) extending through the balloon, the coil having a coil interior and a coil exterior;
a polymer coating (58) disposed on the coil interior; and
a guidewire port (48) disposed within the distal region, proximal of the balloon;
wherein the coil extends through the balloon and to the guidewire port, thereby forming a guidewire lumen extending from the guidewire port to the distal end of the catheter.

2. The balloon catheter of claim 1, further comprising a heat shrink polymer disposed about the coil exterior.

3. The balloon catheter of claim 1, wherein the coil is disposed within the shaft lumen.

4. The balloon catheter of claim 1, the coil interior providing an extension of the guidewire lumen.

5. The balloon catheter of claim 1, wherein the coil extends proximally of the balloon.

6. The balloon catheter of claim 1, wherein the coil comprises a coil wire, and the polymer coating is disposed on the coil by coating the coil wire before coiling the coil wire to form the coil.

7. The balloon catheter of claim 6, wherein the coil wire is coated with polytetrafluoroethylene.

8. The balloon catheter of claim 6, wherein the coil wire comprises a flat ribbon prior to being coiled.

## Patentansprüche

1. Ballonkatheder (52), der aufweist:
einen Längsschaft (28), der einen distalen Abschnitt (54) hat, der ein distales Ende definiert, wobei ein Schaftlumen durch den Längsschaft zum distalen Ende verläuft;
einen Ballon (30), der um den distalen Abschnitt des Längsschafts angeordnet ist;
eine Spule (56), die durch den Ballon verläuft, wobei die Spule eine Spuleninnenseite und eine Spulenaußenseite aufweist;
eine Polymerbeschichtung (58), die an der Spuleninnenseite angeordnet ist; und
eine Führungsdrahtöffnung (48), die am distalen Abschnitt proximal zum Ballon angeordnet ist;
wobei die Spule durch den Ballon und zur Führungsdrahtöffnung verläuft, wodurch ein Führungsdrahtlumen gebildet wird, das von der Führungsdrahtöffnung zum distalen Ende des Katheters verläuft.

2. Ballonkatheder nach Anspruch 1, der weiterhin ein Wärmeschrumpfpolymer aufweist, das um die Spulenaußenseite angeordnet ist.

3. Ballonkatheder nach Anspruch 1, wobei die Spule im Schaftlumen angeordnet ist.

4. Ballonkatheder nach Anspruch 1, wobei die Spuleninnenseite eine Erweiterung des Führungsdrahtlumens bereitstellt.

5. Ballonkatheder nach Anspruch 1, wobei sich die Spule proximal zum Ballon erstreckt.

6. Ballonkatheder nach Anspruch 1, wobei die Spule einen Spulendraht aufweist, und die Polymerbeschichtung auf der Spule angeordnet ist, indem der Spulendraht vor dem Wickeln des Spulendrahts beschichtet wird, um die Spule zu bilden.

7. Ballonkatheder nach Anspruch 6, wobei der Spulendraht mit Polytetrafluorethylen beschichtet ist.

8. Ballonkatheder nach Anspruch 6, wobei der Spulendraht vor der Wicklung ein flaches Band aufweist.

## Revendications

1. Cathéter à ballonnet (52) comprenant :
une tige allongée (28) ayant une région distale (54) définissant une extrémité distale, une lumière de tige s'étendant à travers la tige allongée jusqu'à l'extrémité distale ;
un ballonnet (30) disposé autour de la région distale de la tige allongée ;
une bobine (56) s'étendant à travers le ballonnet, la bobine ayant un intérieur hélicoïdal et un extérieur hélicoïdal ;
un revêtement polymère (58) disposé sur l'intérieur hélicoïdal ; et
un orifice de fil-guide (48) disposé à l'intérieur de la région distale, à proximité du ballonnet ;
dans lequel la bobine s'étend à travers le ballonnet et jusqu'à l'orifice de fil-guide, formant ainsi une lumière de fil-guide s'étendant de l'orifice de fil-guide jusqu'à l'extrémité distale du cathéter.

2. Cathéter à ballonnet selon la revendication 1, comprenant en outre un polymère thermorétractable disposé autour de l'extérieur hélicoïdal.

3. Cathéter à ballonnet selon la revendication 1, dans lequel la bobine est disposée à l'intérieur de la lumière de tige.

4. Cathéter à ballonnet selon la revendication 1, l'intérieur hélicoïdal fournissant une extension de la lumière de fil-guide.

5. Cathéter à ballonnet selon la revendication 1, dans lequel la bobine s'étend à proximité du ballonnet.

6. Cathéter à ballonnet selon la revendication 1, dans lequel la bobine comprend un fil de bobine, et le revêtement polymère est disposé sur la bobine en recouvrant le fil de bobine avant d'enrouler le fil de bobine afin de former la bobine.

7. Cathéter à ballonnet selon la revendication 6, dans lequel le fil de bobine est recouvert avec du polytétrafluoroéthylène.

8. Cathéter à ballonnet selon la revendication 6, dans lequel le fil de bobine comprend un ruban plat avant d'être enroulé.
